# EUROPEAN PATENT APPLICATION

(11) **EP 2 332 908 A1**
(43) Date of publication of application: **15.06.2011**
(21) Application number: 09814568.3
(22) Date of filing: 15.09.2009
(51) Int. Cl.: C07D 207/16

(54) **METHOD FOR PURIFYING AMINOACETYLPYRROLIDINECARBONITRILE DERIVATIVE AND SALT THEREOF**

(30) Priority: 16.09.2008 JP 2008235978
(71) Applicant: Kyorin Pharmaceutical Co., Ltd., Tokyo 101-8311 (JP)
(72) Inventor: SHIGA Futoshi, Shimotsuga-gun Tochigi 329-0114 (JP)
(74) Representative: Vossius & Partner
(86) International application number: PCT/JP2009/066091
(87) International publication number: WO 2010/032723

(57) **Abstract**

The present invention herein provides a simple, efficient and industrially useful method for purifying an aminoacetylpyrrolidinecarbonitrile derivative which is useful as a DPP-IV inhibitor. The method comprises the steps of acting an acid such as maleic acid on an aminoacetylpyrrolidinecarbonitrile derivative represented by the following Formula 1 to form a salt, isolating the salt and then regenerating the foregoing derivative from the salt: 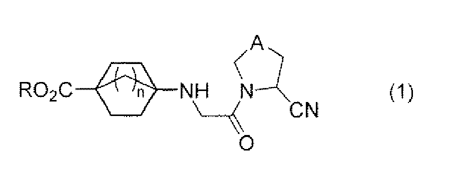 (In the formula, A represents CH₂, CHF or CF₂; R represents a C₁ to C₆ alkyl group which may have a substitutent, a C₃ to C₈ cycloalkyl group which may have a substitutent, an arylmethyl group which may have a substitutent, an arylethyl group which may have a substitutent, an aromatic hydrocarbon ring which may have a substitutent, an aromatic hetero ring which may have a substitutent, or an aliphatic hetero ring which may have a substitutent; and n represents 1 or 2).

## Description

### Technical Field

The present invention relates to a salt of an aminoacetylpyrrolidinecarbonitrile derivative suitable for the purification of an aminoacetylpyrrolidinecarbonitrile derivative useful for the prevention and/or the treatment of diseases associated with dipeptidyl peptidase IV (hereunder referred to as "DPP-IV"), as well as a method for purifying an aminoacetylpyrrolidinecarbonitrile derivative using the salt.

### Background Art

In recent years, a DPP-IV-inhibitor has become of major interest as a drug for treating diabetes (in particular, type II diabetes) and there have been reported a large number of derivatives each showing a DPP-IV inhibitor. For instance, the applicant of this patent application already proposed an aminoacetylpyrrolidinecarbonitrile derivative represented by the following structural formula (Formula 1) and a method for preparing the derivative (Patent Documents 1 to 2).

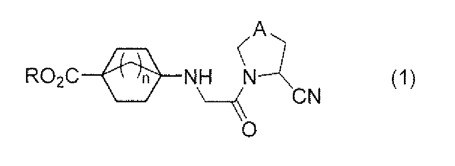

(Wherein A represents CH₂, CHF or CF₂;
R represents a C₁ to C₆ alkyl group which may have a substitutent, a C₃ to C₈ cycloalkyl group which may have a substitutent, an arylmethyl group which may have a substitutent, an arylethyl group which may have a substitutent, an aromatic hydrocarbon ring which may have a substitutent, an aromatic hetero ring which may have a substitutent, or an aliphatic hetero ring which may have a substitutent; and
n represents 1 or 2).
However, these Patent Documents 1 and 2 simply disclose purification methods such as the crystallization technique in which water is added to the reaction solution, and the silica gel chromatography technique using ethyl acetate, methanol or the like as an eluting solvent.

### Prior Art Literature

### Patent Document

Patent Document 1: WO 2005/075421 Pamphlet
Patent Document 2: WO 2007/102286 Pamphlet

### Summary of the Invention

### Problem That the Invention is to Solve

It is an object of the present invention to provide a method for simply and efficiently purifying an aminoacetylpyrrolidinecarbonitrile derivative which is useful as a DPP-IV inhibitor and therefore, to provide an industrially useful purification method.

### Means for the Solution of the Problems

The inventors of this invention have conducted intensive studies on a method for purifying aminoacetylpyrrolidinecarbonitrile derivatives, have found that such an aminoacetylpyrrolidinecarbonitrile derivative can simply and efficiently be purified by acting a specific kind of an organic acid or inorganic acid on an aminoacetylpyrrolidinecarbonitrile derivative represented by the foregoing Formula 1 to thus once form a salt of the aminoacetylpyrrolidinecarbonitrile derivative, purifying the resulting salt of the aminoacetylpyrrolidinecarbonitrile derivative and then acting a base on the purified salt of the aminoacetylpyrrolidinecarbonitrile derivative, and have thus completed the present invention.

According to the present invention, a salt of an aminoacetylpyrrolidinecarbonitrile derivative is isolated, the salt is subjected to a desalting treatment using a base and the resulting desalted product is then recrystallized, thereby, it is possible to improve the efficiency of purifying such a derivative and to remove the impurities whose removal had been quite difficult.
Accordingly, the present invention relates to the following inventions:

1. A method for purifying an aminoacetylpyrrolidinecarbonitrile derivative comprising steps of:
   (1) a step of acting maleic acid, an organic sulfonic acid or an inorganic acid on an aminoacetylpyrrolidinecarbonitrile derivative represented by the following general formula 1 to thereby form a salt of the aminoacetylpyrrolidinecarbonitrile derivative: 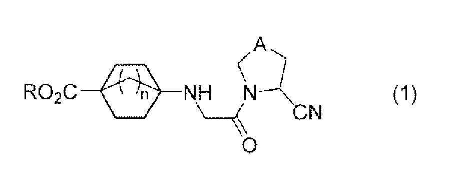
   wherein
   A represents CH₂, CHF or CF₂;
   R represents a C₁ to C₆ alkyl group which may have a substitutent, a C₃ to C₈ cycloalkyl group which may have a substitutent, an arylmethyl group which may have a substitutent, an arylethyl group which may have a substitutent, an aromatic hydrocarbon ring which may have a substitutent, an aromatic hetero ring which may have a substitutent, or an aliphatic hetero ring which may have a substitutent; and
   n represents 1 or 2;

(2) a step of isolating the salt of the aminoacetylpyrrolidinecarbonitrile derivative: and
(3) a step of treating the isolated salt of the aminoacetylpyrrolidinecarbonitrile derivative with a base to thus bring the salt back to the aminoacetylpyrrolidinecarbonitrile derivative.

2. A salt of an aminoacetylpyrrolidinecarbonitrile derivative represented by the following general formula 2: 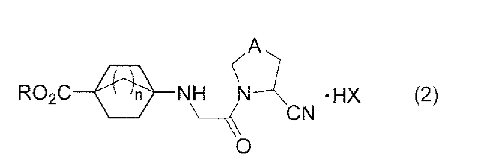 wherein
   A represents CH₂, CHF or CF₂;
   R represents a C₁ to C₆ alkyl group which may have a substitutent, a C₃ to C₈ cycloalkyl group which may have a substitutent, an arylmethyl group which may have a substitutent, an arylethyl group which may have a substitutent, an aromatic hydrocarbon ring which may have a substitutent, an aromatic hetero ring which may have a substitutent, or an aliphatic hetero ring which may have a substitutent;
   n represents 1 or 2; and
   HX represents maleic acid, an organic sulfonic acid or an inorganic acid.

3. An aminoacetylpyrrolidinecarbonitrile derivative represented by the following general formula 1 and characterized in that the derivative has an impurity content of less than 0.1%: 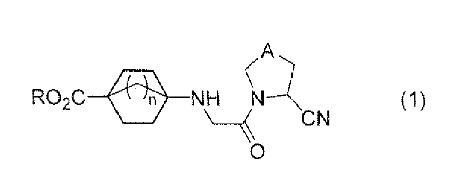 wherein
   A represents CH₂, CHF or CF₂;
   R represents a C₁ to C₆ alkyl group which may have a substitutent, a C₃ to C₅ cycloalkyl group which may have a substitutent, an arylmethyl group which may have a substitutent, an arylethyl group which may have a substitutent, an aromatic hydrocarbon ring which may have a substitutent, an aromatic hetero ring which may have a substitutent, or an aliphatic hetero ring which may have a substitutent; and
   n represents 1 or 2.

### Effects of the Invention

The purification methods disclosed in Patent Documents 1 and 2 suffer from problems in that the quality of the resulting product is low and that the production cost is high since, for instance, the methods are insufficient in the degree of purification and they require the use of complicated operations for the purification. Contrary to this, the purification method according to the present invention permits the simple and efficient preparation of an aminoacetylpyrrolidinecarbonitrile derivative having a high degree of purity and therefore, the method would considerably be useful from the industrial standpoint.

### Mode for Carrying Out the Invention

The expression "C₁ to C₆ alkyl group which may have a substituent" used herein means a C₁ to C₆ alkyl group which may have to 5 substituents selected from the group consisting of halogen atoms, a hydroxyl group, a cyano group, C₁ to C₆ alkoxy groups, aryloxy groups which may have a substituent, C₁ to C₆ alkylcarbonyl groups, C₁ to C₆ alkoxycarbonyl groups, C₁ to C₆ alkylthio groups, an amino group, mono- or di-substituted C₁ to C₆ alkylamino groups, 4- to 9-membered cyclic amino groups which may contain 1 to 3 hetero atoms, a formylamino group, C₁ to C₆ alkylcarbonylamino groups, C₁ to C₆ alkoxycarbonylamino groups, C₁ to C₆ alkylsulfonylamino groups, and arylsulfonylamino groups which may have a substituent.
The term "C₁ to C₆ alkyl group" used herein means a linear or branched lower alkyl group having 1 to 6 carbon atoms and specific examples thereof include a methyl group, an ethyl group, a propyl group, a 1-methylethyl group, a 1-methylpropyl group, a 2-methylpropyl group, a 1-ethylpropyl group, a 2-ethylpropyl group, a butyl group and a hexyl group.

The expression "C₃ to C₈ cycloalkyl group which may have a substituent" used herein means a C₃ to C₈ cycloalkyl group which may have 1 to 5 substituents selected from the group consisting of halogen atoms, a hydroxyl group, a cyano group, C₁ to C₆ alkoxy groups, aryloxy groups which may have a substituent, C₁ to C₆ alkylcarbonyl groups, C₁ to C₆ alkoxycarbonyl groups, C₁ to C₆ alkylthio groups, an amino group, mono- or di-substituted C₁ to C₆ alkylamino groups, 4- to 9-membered cyclic amino groups which may contain 1 to 3 hetero atoms, a formylamino group, C₁ to C₆ alkylcarbonylamino groups, C₁ to C₆ alkoxycarbonylamino groups, C₁ to C₆ alkylsulfonylamino groups, and arylsulfonylamino groups which may have a substituent.
The term "C₃ to C₈ cycloalkyl group" used herein means an alkyl group including a cycloalkyl ring and having 3 to 8 carbon atoms and specific examples thereof are a cyclopropyl group, a cyclopropylmethyl group, a cyclobutyl group, a cyclopentyl group, a cyclohexyl group, a cycloheptyl group, and a cyclooctyl group.

Moreover, the term "substituent" appearing in the foregoing expression "aryloxy group which may have a substituent" or "arylsulfonylamino group which may have a substituent" has the same meaning specified in connection with that appearing in the expression "arylmethyl group which may have a substituent" as will be described below. In addition, the term "aryl group" appearing therein has the same meaning as that appearing in the expression "arylmethyl group which may have a substituent." The same rule will apply correspondingly to the following description unless otherwise clearly specified. The same is true for the expression "arylamino group which may have a substituent" as will be referred to below.

Further, the meaning of the term "C₁ to C₆ alkyl group" is the same as that defined above even in the group containing C₁ to C₆ alkyl group such as "C₁ to C₆ alkoxy group," "C₁ to C₆ alkylcarbonyl group," "C₁ to C₆ alkoxycarbonyl group," "C₁ to C₆ alkylthio group," "mono- or di-substituted C₁ to C₆ alkylamino group," "C₁ to C₆ alkylcarbonylamino group," "C₁ to C₆ alkoxycarbonylamino group," and "C₁ to C₆ alkylsulfonylamino group." This term will also be so defined that it has the same meaning even in the following description unless otherwise specified.

The expression "arylmethyl group which may have a substituent" used in this specification means an arylmethyl group (for example, the specific examples thereof include a phenylmethyl group, a naphthylmethyl group, a pyridylmethyl group, a quinolylmethyl group, an indolylmethyl group and the like) which may have 1 to 5 substituents selected from the group consisting of halogen atoms, C₁ to C₆ alkyl groups which may have a substituent, a hydroxyl group, a cyano group, a nitro group, C₁ to C₆ alkoxy groups which may have a substituent, aryloxy groups which may have a substituent, C₁ to C₆ alkylcarbonyl groups, C₁ to C₆ alkoxycarbonyl groups, C₁ to C₆ alkylthio groups, an amino group, C₁ to C₆ alkylamino groups which may be mono- or di-substituted, arylamino groups which may have a substituent, 4-to 9-membered cyclic amino groups which may contain 1 to 3 hetero atoms, formylamino group, C₁ to C₆ alkylcarbonylamino groups, C₁ to C₆ alkoxycarbonylamino groups, C₁ to C₆ alkylsulfonylamino groups, and arylsulfonylamino groups which may have a substituent.

The expression "arylethyl group which may have a substituent" used in this specification means an arylethyl group (for example, the specific examples thereof include a phenylethyl group, a naphthylethyl group, a pyridylethyl group, a quinolylethyl group, an indolylethyl group and the like) which may have 1 to 5 substituents selected from the group consisting of halogen atoms, C₁ to C₆ alkyl groups which may have a substituent, a hydroxyl group, a cyano group, a nitro group, C₁ to C₆ alkoxy groups which may have a substituent, aryloxy groups which may have a substituent, C₁ to C₆ alkylcarbonyl groups, C₁ to C₆ alkoxycarbonyl groups, C₁ to C₆ alkylthio groups, an amino group, C₁ to C₆ alkylamino groups which may be mono- or di-substituted, arylamino groups which may have a substituent, 4-to 9-membered cyclic amino groups which may contain 1 to 3 hetero atoms, formylamino group, C₁ to C₆ alkylcarbonylamino groups, C₁ to C₆ alkoxycarbonylamino groups, C₁ to C₆ alkylsulfonylamino groups, and arylsulfonylamino groups which may have a substituent.
In this connection, the meaning of the term "substituent" appearing in the expression "C₁ to C₆ alkoxy group which may have a substituent" is the same as that specified above in connection with the expression "C₁ to C₆ alkyl group which may have a substituent."

The expression "aromatic hydrocarbon ring which may have a substituent" used in this specification means an aromatic hydrocarbon ring (specific examples thereof include a benzene ring, a naphthalene ring or an anthracene ring) which may have 1 to 5 substituents selected from the group consisting of halogen atoms, a hydroxyl group, a cyano group, a nitro group, C₁ to C₆ alkyl groups which may have a substituent, C₁ to C₆ alkoxy groups, C₁ to C₆ alkylthio groups, and C₁ to C₆ dialkylamino groups.

The expression "aromatic hetero ring which may have a substituent" used in this specification means an aromatic hetero ring which may have 1 to 5 substituents selected from the group consisting of halogen atoms, a hydroxyl group, a cyano group, a nitro group, C₁ to C₆ alkyl groups, C₁ to C₆ alkoxy groups, and C₁ to C₆ alkylthio groups (for instance, 5- or 6-membered aromatic monocyclic hetero rings, or 9- or 10-membered aromatic fused heterocyclic rings, which have 1 to 3 hetero atoms arbitrarily selected from among nitrogen, oxygen and sulfur atoms, such as a pyridine ring, a pyrimidine ring, a pyridazine ring, a triazine ring, a quinoline ring, a naphthyridine ring, a quinazoline ring, an acridine ring, a pyrrole ring, a furan ring, a thiophene ring, an imidazole ring, a pyrazole ring, an oxazole ring, an isoxazole ring, a thiazole ring, an indole ring, a benzofuran ring, a benzothiazole ring, a benzimidazole ring or a benzoxazole ring).

The expression "aliphatic hetero ring which may have a substituent" used in this specification means an aliphatic hetero ring (4- to 7-membered aliphatic monocyclic hetero rings or 9- or 10-membered aliphatic fused heterocyclic rings, which have 1 to 3 hetero atoms arbitrarily selected from among nitrogen, oxygen and sulfur atoms, such as an azetidine ring, a pyrrolidine ring, a tetrahydrofuran ring, a piperidine ring, a morpholine ring or a piperazine ring) which may have 1 to 5 substituents selected from the group consisting of halogen atoms, C₁ to C₆ alkyl groups, a hydroxyl group, a cyano group, a nitro group, C₁ to C₆ alkoxy groups, and C₁ to C₆ alkylthio groups.

The term "acid" used in this specification means an organic acid or an inorganic acid. Examples of such organic acids include organic carboxylic acids such as propionic acid, citric acid, maleic acid, and benzoic acid; and organic sulfonic acids such as methanesulfonic acid, ethanesulfonic acid, propanesulfonic acid, 2-propanesulfonic acid, benzenesulfonic acid, 4-toluenesulfonic acid, and naphthalenesulfonic acid. Examples of such inorganic acids are hydrochloric acid, sulfuric acid, nitric acid, phosphoric acid, phosphorous acid, hydrobromic acid, carbonic acid and the like. Among them, preferred are maleic acid, organic sulfonic acids and hydrochloric acid as the acids providing excellent crystallizability and high purification effect, and more preferably used herein are maleic acid or methanesulfonic acid.

The present invention will hereunder be described in more detail.
The aminoacetylpyrrolidinecarbonitrile derivative represented by the general formula 1 and used in the present invention can be prepared by the use of, for instance, the method disclosed in Patent Document 2. More specifically, the aminoacetylpyrrolidinecarbonitrile derivative represented by the general formula 1 can be prepared by subjecting a sulfonyloxyacetylpyrrolidinecarbonitrile derivative represented by the following general formula 3:

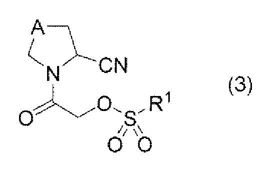

(wherein R¹ represents a C₁ to C₆ alkyl group which may have a substituent, a C₃ to C₈ cycloalkyl group which may have a substituent, an arylmethyl group which may have a substituent, an aromatic hydrocarbon ring which may have a substituent, an aromatic hetero ring which may have a substituent or an aliphatic hetero ring which may have a substituent; and A is the same as that defined above), to a reaction, in the presence or absence of a base, with an amine derivative thereof represented by the following general formula 4 or a salt:

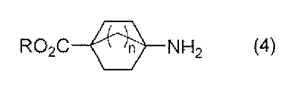

(wherein R and n are the same as those defined above).

When using a base in the foregoing reaction, examples of such bases usable herein include alkali metal carbonates such as sodium hydrogen carbonate, potassium carbonate or cesium carbonate; and tertiary amines such as triethylamine, diisopropylethylamine, N-methylmorpholine, diazabicyclo[.5.4.0]-7-undecene, pyridine, 4-dimethylaminopyridine, 1,8-bis(dimethylamino) naphthalene, phosphazene base or pentaisopropylguanidine, with potassium carbonate being preferably used herein. In case where a catalyst is used in this reaction, a phase transfer catalyst or an inorganic salt such as tetrabutylammonium bromide, tetrabutylammonium iodide, benzyltriethylammonium bromide, lithium bromide, lithium iodide, sodium iodide, potassium bromide, potassium iodide, cesium bromide and cesium iodide; with potassium iodide being preferably used in the present invention. As a solvent used in this reaction, usable in the present invention may be, for instance, inert ones, which do not take part in the reaction, such as acetone, ethanol, tetrahydrofuran, dioxane, ethyl ether, dimethoxyethane, acetonitrile, ethyl acetate, toluene, xylene, dichloromethane, chloroform, 1,2-dichloroethane, N,N-dimethylformamide, N,N-dimethylacetamide, N-methyl-2-pyrrolidone, and dimethyl sulfoxide, with NN-dimethylformamide being preferably used. The reaction can be carried out at a temperature ranging from 30 to 150°C and preferably 0 to 50°C.

### (Purification Method)

The purification method according to the present invention is one which makes use of an organic acid salt or an inorganic acid salt of an aminoacetylpyrrolidinecarbonitrile derivative represented by the foregoing general formula 2 as an intermediate. Thus, the aminoacetylpyrrolidinecarbonitrile derivative can be purified by repeating the crystallization and separation or isolation of the same through the formation of an organic acid salt or an inorganic acid salt of the aminoacetylpyrrolidinecarbonitrile derivative as an intermediate (see Reaction Scheme 1 given below).

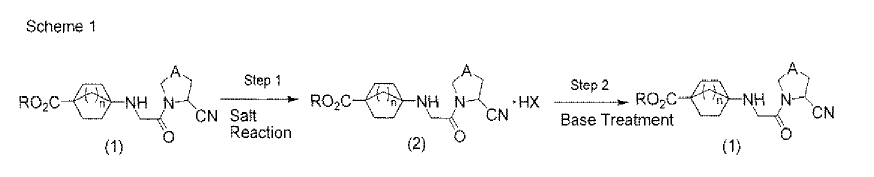

(In the reaction scheme 1, A, R, n and HX are the same as those specified above.)

The step 1 is one in which an aminoacetylpyrrolidinecarbonitrile derivative represented by the foregoing general formula 1 is reacted with an acid to form a salt of the aminoacetylpyrrolidinecarbonitrile derivative represented by the foregoing general formula 2. This step permits the preparation of the intended salt of the aminoacetylpyrrolidinecarbonitrile derivative by carrying out the crystallization and the isolation of the salt of the derivative.
As the acid used in this step 1, there can be listed, for instance, organic carboxylic acids such as propionic acid, citric acid, maleic acid, and benzoic acid; organic sulfonic acids such as methanesulfonic acid, ethanesulfonic acid, propanesulfonic acid, 2-propanesulfonic acid, benzenesulfonic acid, 4-toluene sulfonic acid, and naphthalenesulfonic acid; and inorganic acids such as hydrochloric acid, sulfuric acid, nitric acid, phosphoric acid, phosphorous acid, hydrobromic acid and carbonic acid. Among them, preferably used in this step are maleic acid, organic sulfonic acids and hydrochloric acid as the acids providing excellent crystallizability and high purification effect, and more preferably used herein are maleic acid and methanesulfonic acid. The amount thereof to be used is preferably not less than one equivalent and more preferably 1 to 1.1 equivalents relative to the amount of the compound represented by the foregoing general formula 2.

The solvent used in the step 1 may be an inert one which does not take part in the reaction and specific examples thereof include protic solvents such as water, ethanol, and 2-propanol; ethers such as tetrahydrofuran, dioxane and dimethoxy ethane; esters such as ethyl acetate; ketones such as acetone; and hydrocarbons such as benzene, toluene and xylene. Examples of solvent showing a high purification effect include ethanol, 2-propanol and acetone, with acetone being particularly preferably used in this step. In addition, these solvents may be used alone or in a mixture thereof.
The foregoing salt-forming reaction may be carried out at a temperature ranging from room temperature to the boiling point of the solvent used, preferably 30°C to the boiling point of the solvent used and particularly preferably 40°C to the boiling point of the solvent used.
The salt of the aminoacetylpyrrolidinecarbonitrile derivative represented by the foregoing formula 2 and thus prepared is subsequently crystallized from the reaction solvent and then isolated.

The step 2 is one wherein the isolated salt of the aminoacetylpyrrolidinecarbonitrile derivative is treated with a base to form an aminoacetylpyrrolidinecarbonitrile derivative represented by the foregoing general formula 1.
As such a base used in this step, there may be listed, for instance, hydroxides such as lithium hydroxide, sodium hydroxide and potassium hydroxide; carbonates such as sodium carbonate, potassium carbonate and sodium hydrogen carbonate; inorganic bases such as ammonia; and organic bases such as triethylamine, and pyridine and preferably used herein are sodium carbonate and potassium carbonate. The amount thereof to be used is preferably not less than one equivalent and more preferably 1 to 2 equivalents relative to the amount of the compound represented by the general formula 2.

The solvent used in this step is not restricted to any particular one inasmuch as it is an inert solvent which does not take part in the reaction at all and specific examples thereof are protic solvents such as water, ethanol and 2-propanol; ethers such as tetrahydrofuran and dioxane; and acetone, with water being preferably used herein. Moreover, these solvents may be used alone or in a mixture thereof.
The treatment with a base can be carried out at a temperature ranging from 0°C to the boiling point of the solvent used, preferably 0°C to 50°C and particularly preferably 30°C to 50°C.

The aminoacetylpyrrolidinecarbonitrile derivative represented by the foregoing general formula 1 and thus obtained can be crystallized from the reaction solution and then isolated to thus purify the same. Moreover, when the reaction solvent used is an organic solvent, it is also possible to add water to the reaction system so as to crystallize the derivative from the mixed solvent of the organic solvent and water and to thus isolate the resultant product.
According to the method of the present invention, the crystallization and isolation of the derivative are carried out in each step to thus simply and efficiently purify the desired derivative. In addition, the recrystallization of the derivative may be carried out, as the need arises, to further improve the purity of the intended derivative.

### Example

Now, Examples of the present invention will be described below, but the present invention is by no means limited to these specific Examples at all. In this connection, the starting compounds used in the following Examples were prepared according to the methods disclosed in Patent Document 2 and the specific procedures thereof were given below as Reference Examples. Incidentally, the numerical values relating to purity and impurity-contents in the following Examples and Comparative Examples were determined according to the HPLC technique carried out under the following conditions:

### Conditions A for the Measurement:

Detector: An ultraviolet absorptiometer (wavelength used for the measurement: 205 nm);
Column Used: Inertsil ODS-3 (Trade Name; available from GL Science Inc.), 4.6 mm (internal diameter) X 15 cm (length);
Guard Column: Inertsil ODS-3 (Trade Name; available from GL Science Inc.), 4.0 mm (internal diameter) X 10 mm (length);
Column Temperature: 30°C;
Mobile Phase: A 0.1% phosphoric acid aqueous solution containing 5 mmol/L sodium 1-octanesulfonate was used as Liquid A and acetonitrile for liquid chromatography was used as Liquid B. The ratio: Liquid A/Liquid B = 66/34.
Flow Rate: 1.0 mL/min.

### Conditions B for the Measurement:

Detector: An ultraviolet absorptiometer (wavelength used for the measurement: 210 nm);
Column Used: Inertsil ODS-3V (Trade Name; available from GL Science Inc.), 4.6 mm (internal diameter) X 15 cm (length);
Guard Column: Inertsil ODS-3 (Trade Name; available from GL Science Inc.), 4.0 mm (internal diameter) X 10 mm (length);
Column Temperature: 30°C;
Mobile Phase: Liquid A/liquid B = 80/20→60/40;
Flow Rate: 1.0 mL/min.

### (Reference Example 1): Synthesis of (2S,4S)-1-[2-[(4-ethoxycarbonylbicyclo[2.2.2] oct-1-yl)amino] acetyl]-4-fluoropyrrolidine-2-carbonitrile:

13.2 g of ethyl 4-aminobicyclo[2.2.2]octane-1-carboxylate trifluoroacetate was dissolved in 72 mL of N,N-dimethylformamide, followed by the addition of 11.7 g of potassium carbonate to the resulting solution and the stirring of the mixture at an internal temperature of 40°C for 20 minutes. Then, 638 mg of potassium iodide and 12.0 g of (2S,4S)-4-fluoro-1-[2-(benzenesulfonyloxy)acetyl]pyrrolidine-2-carbonitrile were added to the stirred mixture, and the resulting mixture was stirred for additional 5 hours. Water (216 mL) was added to the reaction solution and the resulting mixture was stirred at an internal temperature of not higher than 10°C for one hour. The crystals thus precipitated were collected by filtration, washed with 144 mL of water, dried at 60°C while blowing air on the crystals to thus give the title compound as white crystals (yield: 10.1 g (75%)).

### (Reference Example 2): Synthesis of (2S,4S)-1-[2-[(4-ethoxycarbonylbicyclo[2.2.2] oct-1-yl)amino]acetyl-4-fluoropyrrolidine-2-carbonitrile:

After dissolving ethyl 4-aminobicyclo[2.2.2]octane-1-carboxylate trifluoroacetate (76.7 g) in N,N-dimethylformamide (245 mL), potassium carbonate (68.1 g) and potassium iodide (37.2 g) were added to the resulting solution and the mixture was stirred at an internal temperature ranging from 60 to 67°C for 30 minutes. Then, there was dropwise added a solution of (2S,4S)-4-fluoro-1-(2-(benzenesulfonyloxy)acetyl]pyrrolidine-2-carbonitrile (70.0 g) in N,N-dimethyl formamide (280 mL) to the mixture at an internal temperature ranging from 65 to 66 °C for 2 hours, followed by the washing with N,N-dimethylformamide (35 mL) and the stirring of the mixture at an internal temperature ranging from 64 to 66°C for 30 minutes. Water (1.68 L) was dropwise added to the reaction system at an internal temperature ranging from 55 to 66°C, followed by the cooling of the mixture, and the stirring of the mixture at an internal temperature ranging from 47 to 48°C for 10 minutes and then at an internal temperature ranging from 6 to 15°C for 30 minutes. The crystals thus precipitated were collected by filtration, washed with water (280 mL) and dried under reduced pressure at 60°C to thus obtain the title compound as white crystals (yield: 60.5 g (77%)).

### Example 1: Synthesis of (2S,4S)-1-[2-[(4-ethoxycarbonylbicyclo[2.2.2]oct-1-yl) amino]acetyl]-4-fluoropyrrolidine-2-carbonitrile maleate:

The compound (500 mg) prepared in Reference Example 1 was dissolved, with heating, in 2-propanol (10 mL), a solution of maleic acid (173 mg) in 2-propanol (7 mL) was added to the resulting solution, and the resulting mixture was cooled to room temperature. The crystals thus precipitated were collected by filtration, washed with 2-propanol and dried under reduced pressure at 60°C to thus give the title compound as a colorless powder (yield: 589 mg (89%)).
mp: 213-215°C (decomposed)

### Example 2: Synthesis of (2S,4S)-1-[2-[(4-ethoxycarbonylbicyclo[2.2.2]oct-1-yl) amino]acetyl]-4-fluoropyrrolidine-2-carbonitrile methanesulfonate:

The same procedures used in Example 1 were carried out except for using methanesulfonic acid to thus form the title compound as a colorless powder (yield: 97%).
mp: 233-235°C

### Example 3: Synthesis of (2S,4S)-1-[2- (4-ethoxycarbonylbicyclo[2.2.2]oct-1-yl) amino]acetyl]-4-fluoropyrrolidine-2-carbonitrile 4-toluenesulfonate:

The compound (500 mg) prepared in Reference Example 2 was dissolved in acetone (10 mL), 4-toluenesulfonic acid monohydrate (284 mg) was added to the resulting solution at an external temperature of 50°C and then the resulting mixture was stirred at that temperature for 10 minutes. The mixture was cooled to room temperature and then stirred for additional 30 minutes. The crystals thus precipitated therefrom were collected by filtration, washed with acetone (5 mL) and subsequently dried under reduced pressure at room temperature to thus give the title compound as a colorless powder (yield: 735 mg (99%)).
mp: 246-248°C

### Example 4: Synthesis of (2S,4S)-1-[2-[(4-ethoxycarbonylbicyclo[2.2.2]oct-1-yl) amino]acetyl]-4-fluoropyrrolidine-2-carbonitrile maleate:

The compound (500 mg) prepared in Reference Example 2 was dissolved in acetone (7.5 mL), a solution of maleic acid (177 mg) in acetone (1.5 mL) was dropwise added to the resulting solution at an external temperature of 45°C and then washed with acetone (1 mL). The resulting mixture was stirred at that temperature for 30 minutes, followed by allowing the mixture to cool to room temperature and the stirring thereof for additional 30 minutes. The crystals thus precipitated were collected by filtration, washed with acetone (2.5 mL) and dried under reduced pressure at 50°C to thus form the title compound as a colorless powder (yield: 97%).

### Example 5: Synthesis of (2S,4S)-1-[2-[(4-ethoxycarbonylbicyclo[2.2.2]oct-1-yl) amino]acetyl]-4-fluoropyrrolidine-2-carbonitrile hydrochloride:

The compound (500 mg) prepared in Reference Example 2 was dissolved in acetone (10 mL) and then hydrochloric acid (150 µL) was dropwise added to the resulting solution at an external temperature of 45°C. After stirring the mixture at that temperature for 10 minutes, diisopropyl ether (10 mL) was added to the mixture and the resulting mixture was stirred for additional 20 minutes. The mixture was cooled back to room temperature, stirred for additional 30 minutes, the crystals thus precipitated were collected by filtration, washed with an acetone-diisopropyl ether mixed solvent (1:2, 5 mL) and finally dried under reduced pressure at 50°C to thus obtain the title compound as a colorless powder (yield: 551 mg, quantitative).
mp: 195-197°C (decomposed)

### Example 6: Synthesis of (2S,4S)-1-[2-[(4-ethoxycarbonylbicyclo[2.2.2]oct-1-yl) amino]acetyl]-4-fluoropyrrolidine-2-carbonitrile sulfate:

The compound (500 mg) prepared in Reference Example 2 was dissolved in acetone (10 mL), sulfuric acid (91.0 µL) was dropwise added to the resulting solution at an external temperature of 50°C and then the resulting mixture was cooled back to room temperature. Diisopropyl ether (20 mL) was added to the mixture and the resulting mixture was stirred at room temperature for 30 minutes. The crystals thus precipitated were collected by filtration, washed with an acetone-diisopropyl ether mixed solvent (1:2, 10 mL) and then dried at room temperature under reduced pressure to thus obtain the title compound as a colorless powder (yield: 651 mg, quantitative).
mp: 138-140°C

### Example 7: Synthesis of (2S,4S)-1-[2-[(4-ethoxycarbonylbicyclo[2.2.2]oct-1-yl) amino]acetyl]-4-fluoropyrrolidine-2-carbonitrile hydrobromide:

The compound (500 mg) prepared in Reference Example 2 was dissolved in acetone (10 mL), a 48% hydrobromic acid solution (200 µL) was dropwise added to the resulting solution at an external temperature of 50°C and then the resulting mixture was cooled back to room temperature. Diisopropyl ether (20 mL) was added to the mixture and the resulting mixture was stirred at room temperature for 30 minutes. The crystals thus precipitated were collected by filtration, washed with an acetone-diisopropyl ether mixed solvent (1:2, 15 mL) and then dried at room temperature under reduced pressure to thus obtain the title compound as a colorless powder (yield: 610 mg (99%)).
mp: 174-176°C

### Example 8: Synthesis of (2S,4S)-1-[2-[(4-ethoxycarbonylbicyclo[2.2.2]oct-1-yl) amino]acetyl]-4-fluoropyrrolidine-2-carbonitrile phosphate:

The compound (500 mg) prepared in Reference Example 2 was dissolved in acetone (10 mL), phosphoric acid (120 µL) was dropwise added to the resulting solution at an external temperature of 50°C and then the resulting mixture was cooled back to room temperature. Diisopropyl ether (20 mL) was added to the mixture and the resulting mixture was stirred at room temperature for 30 minutes. The crystals thus precipitated were collected by filtration, washed with an acetone-diisopropyl ether mixed solvent (1:2, 15 mL) and then dried at room temperature under reduced pressure to thus obtain the title compound as a colorless powder (yield: 631 mg (99%)).
mp: 212-214°C

### Example 9: Purification of (2S,4S)-1-[2-[(4-ethoxycarbonylbicyclo[2.2.2]oct-1-yl) amino] acetyl]-4-fluoropyrrolidine-2-carbonitrile (Purification Method 1):

The compound (4.00 g) prepared in Reference Example 1 was dissolved, with heating, in acetone (50 mL), a solution of methanesulfonic acid (1.15 g) in acetone (16 mL) was added to the resulting solution and the resulting mixture was cooled to room temperature. The crystals thus precipitated were collected by filtration, washed with acetone (16 mL) and then dried at 50°C under reduced pressure to thus give the intended methanesulfonic acid salt as a colorless powder (yield: 4.90 g (96%)).
The resulting methanesulfonic acid salt (4.80 g) was dissolved in water (29 mL) and potassium carbonate (2.22 g) was added to the resulting solution gradually in small portions at an internal temperature of 40°C. The reaction solution was cooled by ice water and the crystals thus precipitated were collected by filtration. After washing the crystals with water (14.4 mL), they were dried at 60°C under reduced pressure to thus obtain (2S,4S)-1-[2-[(4-ethoxycarbonylbicyclo[2.2.2]oct-1-yl)amino]acetyl]-4-fluoropyrrolidine-2-carbonitrile as a colorless powder (yield: 3.64 g (97%)).

The resulting (2S,4S)-1-[2-[(4-ethoxycarbonylbicyclo[2.2.2]oct-1-yl)amino] acetyl]-4-fluoropyrrolidine-2-carbonitrile (3.54 g) was dissolved, with heating, in an ethanol-water mixed solvent (4:1, 8.9 mL) and then the resulting solution was allowed to cool to room temperature. After the solution was further cooled by ice water, the crystals thus precipitated were collected by filtration, washed with an ethanol-water mixed solvent (1:1, 7 mL) and dried at 60°C under reduced pressure to thus obtain highly pure (2S,4S)-1-[2-[(4-ethoxycarbonylbicyclo[2.2.2]oct-1-yl)aminolacetyl]-4-fluoropyrrolidi ne-2-carbonitrile (yield: 2.91 g (82%)). Purity: 99.93% (as determined by the HPLC technique under the measuring condition A).

### Example 10: Purification of (2S,4S)-1-[2-[(4-ethoxycarbonylbicyclo[2.2.2]-oct-1-yl) amino]acetyl]-4-fluoropyrrolidine-2-carbonitrile (Purification Method 2):

The compound (20.0 g) prepared in Reference Example 2 was suspended in an acetone-ethanol mixed solvent (4:1, 100 mL) and then hydrochloric acid (5.69 mL) was added to the suspension. After dissolving the compound with heating, 200 mL of diisopropyl ether was dropwise added to the resulting solution at an internal temperature ranging from 41 to 46°C. After stirring the resulting mixture at an internal temperature ranging from 43 to 46°C for 30 minutes, it was cooled and stirred at an internal temperature ranging from 26 to 30°C for 30 minutes. The crystals thus precipitated were collected by filtration, washed with an acetone-diisopropyl ether mixed solvent (1:2, 120 mL) to thus give wet hydrochloride salt.

The resulting wet hydrochloride salt were dissolved, with heating, in water (158 mL), a solution of sodium carbonate (4.48 g) in water (40 mL) was dropwise added to the resulting solution at an internal temperature ranging from 46 to 48°C and the resulting mixture was then stirred at an internal temperature ranging from 47 to 48°C for 30 minutes. The mixture was cooled and stirred at an internal temperature ranging from 24 to 30°C for 30 minutes. The crystals thus precipitated were collected by filtration, washed with water (79 mL), dried at 60°C while blowing air on the crystals to thus give 18.5 g of (2S,4S)-1-[2-[(4-ethoxycarbonyl bicyclo[2.2.2]oct-1-yl)amino]acetyl]-4-fluoropyrrolidine-2-carbonitrile as a colorless powder. After the product was dissolved, with heating, in 80% ethanol (46 mL) (at an internal temperature of 61°C), the insoluble residue were removed by filtration. The filtrate was cooled, stirred at an internal temperature ranging from 36 to 40°C for 10 minutes and further stirred at an internal temperature ranging from 6 to 15°C for 30 minutes. The crystals thus precipitated were collected by filtration, washed with 50% ethanol (18.4 mL), dried at 40°C while blowing air on the crystals to thus give highly pure (2S,4S)-1-[2-[(4-ethoxycarbonylbicyclo[2.2.2]oct-1-yl)amino]acetyl]-4-fluoropyrrolidi ne-2-carbonitrile (yield: 16.3 g, overall yield: 82%). Purity: 99.98% (as determined by the HPLC technique under the measuring condition B).

### Example 11: Purification of (2S,4S)-1-[2-[(4-ethoxycarbonylbicyclo[2.2.2]oct-1-yl) amino]acetyl]-4-fluoropyrrolidine-2-carbonitrile Purification Method 3):

The compound (20.0 g) prepared in Reference Example 2 was dissolved in acetone (300 mL) and then a solution of maleic acid (6.94 g) in acetone (100 mL) was dropwise added to the resulting solution at an internal temperature ranging from 41 to 46°C. After stirring the resulting mixture at an internal temperature ranging from 46 to 48°C for 30 minutes, the mixture was cooled and stirred at an internal temperature ranging from 23 to 30°C for 30 minutes. The crystals thus precipitated were collected by filtration, washed with acetone (100 mL) and dried at 60°C while blowing air on the crystals to thus give the maleic acid salt.

The resulting maleic acid salt was suspended in water (258 mL), hydrochloric acid (11.5 mL) was then added to the suspension and the latter was dissolved with heating. After the addition of water (155 mL) to the solution, a solution of sodium carbonate (17.5 g) in water 103 mL) was dropwise added thereto at an internal temperature ranging from 45 to 49°C and the resulting mixture was stirred at an internal temperature ranging from 45 to 47°C for 30 minutes. The mixture was cooled and stirred at an internal temperature ranging from 24 to 30°C for additional 30 minutes. The crystals thus precipitated were collected by filtration, washed with water (103 mL) and dried at 60°C while blowing air on the crystals to thus give 17.3 g of (2S,4S)-1-[2-[(4-ethoxycarbonylbicyclo[2.2.2]oct-1-yl)amino]acetyl]-4-fluoropyrrolidine-2-carbonitrile as a colorless powder. The product was dissolved, with heating, in 80% ethanol (43 mL) (at an internal temperature of 63°C) and then the insoluble were removed by filtration. The filtrate was cooled, stirred at an internal temperature ranging from 36 to 40°C for 10 minutes and further stirred at an internal temperature ranging from 6 to 15°C for additional 30 minutes. The crystals thus precipitated were collected by filtration, washed with 50% ethanol (17.2 mL) and then dried at 40°C while blowing air on the crystals to thus give highly pure (2S,4S)-1-[2-[(4-ethoxycarbonylbicyclo[2.2.2]oct-1-yl)amino]acetyl]-4-fluoropyrrolidine-2-carbonitrile (yield: 14.9 g; overall yield: 75%). Purity: 99.96% (as determined by the HPLC technique under the measuring condition B).

### (Comparative Examples)

The usefulness of the aminoacetylpyrrolidinecarbonitrile derivatives according to the present invention will be given in the following Table 1 together with the results of Comparative Examples. Moreover, in respect of the effect of the purification method of the present invention, the following Table 2 shows the results obtained by, as Comparative Examples, a purification method comprising only the step of recrystallization and a purification method comprising the treatment of an aminoacetylpyrrolidinecarbonitrile derivative with an acid and/or a base, which has currently been used, and the subsequent recrystallization.

### (Comparative Example 1): Synthesis of (2S,4S)-1-[2-[(4-ethoxycarbonylbicyclo [2.2.2]oct-1-yl)amino]acetyl]-4-fluoropyrrolidine-2-carbonitrile trifluoroacetate:

There were added, to the compound (500 mg) prepared in Reference Example 1, 2-propanol (10 mL) and then trifluoroacetic acid (115 µL), followed by the dissolution of the former with heating, the refrigeration of the resulting solution and the subsequent concentration of the solution under reduced pressure. To the resulting residue, there was added an ethyl acetate-diisopropyl ether mixed solvent (1:5, 6 mL) and the mixture was allowed to stand overnight. The crystals thus precipitated were collected by filtration, washed with an ethyl acetate-diisopropyl ether mixed solvent (1:10, 11 mL) and then dried at 60°C under reduced pressure to thus give the title compound as a colorless powder (yield: 319 mg (48%)).
mp: 158-160°C

### (Comparative Example 2): Synthesis of (2S,4S)-1-[2-[(4-ethoxycarbonylbicyclo [2.2.2]oct-1-yl)amino]acetyl]-4-fluoropyrrolidine-2-carbonitrile fumarate:

The compound (500 mg) prepared in Reference Example 1 was dissolved in acetone (10 mL), fumaric acid (173 mg) was added to the solution at an external temperature of 45°C and then the resulting mixture was stirred at that temperature for 30 minutes (it was found that an agar-like product was formed). The agar-like product was crushed, the crushed product was cooled to room temperature, and it was further stirred for one hour to thus crystallize the same. The crystals thus formed were collected by filtration, washed with acetone (2.5 mL) and dried at 50°C under reduced pressure to thus obtain the title compound as a colorless powder (yield: 680 mg, quantitative).

### (Comparative Example 3): Synthesis of (2S,4S)-1-[2-[(4-ethoxycarbonyl-bicyclo-[2.2.2]-oct-1-yl)-amino]-acetyl]-4-fluoropyrrolidine-2-carbonitrile succinate:

The compound (500 mg) prepared in Reference Example 2 was dissolved in acetone (10 mL), succinic acid (176 mg) was added to the solution at an external temperature of 45 °C and then the resulting mixture was stirred at that temperature for 30 minutes (it was found that an agar-like product was formed). The agar-like product was crushed, acetone (5 mL) was added to the crushed product and the resulting mixture was stirred at room temperature for one hour to thus recrystallize the product. After the addition of acetone (10 mL), the crystals thus precipitated were collected by filtration, washed with acetone (15 mL) and then dried at 50°C under reduced pressure to thus give the title compound as a colorless powder (yield: 495 mg (74%)).

### (Comparative Example 4): Synthesis of (2S,4S)-1-[2-[(4-ethoxycarbonylbicyclo [2.2.2]oct-1-yl)amino]acetyl]-4-fluoropyrrolidine-2-carbonitrile oxalate:

The compound (500 mg) prepared in Reference Example 2 was dissolved in acetone (10 mL), oxalic acid (135 mg) was added to the solution at an external temperature of 45°C (it was found that an agar-like product was formed). The agar-like product was crushed, stirred at that temperature for 30 minutes (the product was crystallized) and further stirred at room temperature for 30 minutes. The crystals thus precipitated were collected by filtration, washed with acetone (2.5 mL) and then dried under reduced pressure at 50°C to thus give the title compound as a colorless powder (yield: 602 mg (96%)).

### (Comparative Example 5): Synthesis of (2S,4S)-1-[2-(4-ethoxycarbonylbicyclo 2.2.2]oct-1-yl)amino]acetyl]-4-fluoropyrrolidine-2-carbonitrile L-tartarate:

The compound (500 mg) prepared in Reference Example 2 was dissolved in acetone (10 mL), L-tartaric acid (224 mg) was added to the solution at an external temperature of 45°C (it was found that an agar-like product was formed). The agar-like product was crushed, stirred at that temperature for 30 minutes (the product was crystallized) and further stirred at room temperature for 30 minutes. The crystals thus precipitated were collected by filtration, washed with acetone (2.5 mL) and then dried at 50°C under reduced pressure to thus give the title compound as a colorless powder (yield: 558 mg (78%)).

### (Comparative Example 6): Synthesis of (2S,4S)-1-[2-[(4-ethoxycarbonylbicyclo [2.2.2]oct-1-yl)amino]acetyl]-4-floropyrrolidine-2-carbonitrile glycolate:

The compound (500 mg) prepared in Reference Example 2 was dissolved in acetone (10 mL), glycolic acid (114 mg) was added to the solution at an external temperature of 45°C. The resulting mixture was stirred at that temperature for 30 minutes, cooled back to room temperature and further stirred for additional 30 minutes. The crystals thus precipitated were collected by filtration, washed with acetone (2.5 mL) and then dried at 50°C under reduced pressure to thus give the title compound as a colorless powder (yield: 503 mg (83%)).

### (Comparative Example 7): Synthesis of (2S,4S)-1-[2-[(4-ethoxycarbonylbicyclo [2.2.2]oct-1-yl)amino]acetyl]-4-fluoropyrrolidine-2-carbonitrile L-lactate:

The compound (500 mg) prepared in Reference Example 2 was dissolved in acetone (10 mL), an 85% L-lactic acid aqueous solution (131 µL) was added to the solution at an external temperature of 45°C. The resulting mixture was stirred at that temperature for 30 minutes, it was allowed to cool to room temperature and further stirred for additional 30 minutes. To the reaction solution, there was added 40 mL of diisopropyl ether and the resulting mixture was further stirred for 30 minutes. The crystals thus precipitated were collected by filtration, washed with an acetone-diisopropyl ether mixed solvent (1:4, 5 mL) and then dried at 50°C under reduced pressure to thus give the title compound as a colorless powder (yield: 504 mg (80%)).

### (Comparative Example 8): Synthesis of (2S,4S)-1-[2-[(4-ethoxycarbonylbicyclo [2.2.2]oct-1-yl)amino]acetyl]-4-fluoropyrrolidine-2-carbonitrile acetate:

The compound (500 mg) prepared in Reference Example 2 was dissolved in acetone (10 mL), acetic acid (85.5 µL) was added to the solution at an external temperature of 45°C. The mixture was stirred at that temperature for 30 minutes, then cooled to room temperature and stirred for additional 30 minutes. To the reaction solution, there was added 40 mL of diisopropyl ether and the resulting mixture was further stirred for 30 minutes. The crystals thus precipitated were collected by filtration, washed with an acetone-diisopropyl ether mixed solvent (1:4, 5 mL) and then dried at 50°C under reduced pressure to thus give the title compound as a colorless powder (yield: 355 mg (61%)).

**[Table 1]**

| Ex. No. | Yield (%) | Crystallizability | Ex. No. | Yield (%) | Crystallizability |
|---|---|---|---|---|---|
| 2 | 97 | Good | 3* | 74 | Agar-like→cryst. |
| 3 | 99 | Good | 4* | 96 | Agar-like→cryst. |
| 4 | 97 | Good | 5* | 78 | Agar-like→cryst. |
| 5 | quantitative | Good | 6* | 83 | Good |
| 1* | 48 | unacceptable | 7* | 80 | unacceptable |
| 2* | quantitative | Agar-like→cryst. | 8* | 61 | unacceptable |

| | | | | | |
|---|---|---|---|---|---|
| *: Comparative Example | | | | | |

As seen from the data listed in Table 1, it is clear that the salts of the aminoacetylpyrrolidinecarbonitrile derivatives prepared in Comparative Examples suffer from a problem in either a yield or crystallizability. Contrary to this, it was found that the salts of the aminoacetylpyrrolidinecarbonitrile derivatives prepared according to the present invention are excellent in both of the yields and crystallizability and that the salts can be prepared in an industrial scale.

### (Comparative Example 9):

The compound (2.00 g) prepared in Reference Example 1 was dissolved, with heating, in an ethanol-water mixed solvent (4:1, 5 mL) and the resulting solution was allowed to cool to room temperature. After further ice-cooling the solution, the crystals precipitated were collected by filtration, washed with an ethanol-water mixed solvent (1:1, 4 mL) and dried at 60°C under reduced pressure to thus give (2S,4S)-1-[2-[(4-ethoxycarbonylbicyclo[2.2.2]oct-1-yl)amino]acetyl]-4-fluoro-pyrrolidine-2-ca rbonitrile (yield: 1.63 g (81%)).

### (Comparative Example 10):

The compound (3.00 g) prepared in Reference Example 1 was dissolved in a 0.5 mol/L hydrochloric acid solution (18 mL) and the resulting solution was washed with ethyl acetate (9 mL). The organic phase was extracted with water (9 mL). The aqueous phases were combined and sodium hydrogen carbonate (1.08 g) was added thereto gradually in small portions at an internal temperature of 40°C. The resulting mixture was stirred at that temperature for one hour, then cooled by water bath and further stirred for one hour. The crystals thus precipitated were collected by filtration, washed with water (21 mL) and then dried at 60°C under reduced pressure to thus obtain (2S,4S)-1-[2-[(4-ethoxycarlaonylbicyclo[2.2.2]oct-1-yl)amino]acetyl]-4-fluoropyrrolidi ne-2-carbonitrile (yield: 2.50 g (83%)).

The resulting (2S,4S)-1-[2-[(4-ethoxycarbonylbicyclo[2.2.2]oct-1-yl)amino] acetyl]-4-fluoropyrrolidine-2-carbonitrile (2.40 g) was dissolved, with heating, in an ethanol-water mixed solvent (4:1, 6 mL) and the resulting solution was allowed to cool to room temperature. After further cooling the solution by ice water, the crystals thus precipitated were collected by filtration, washed with an ethanol-water mixed solvent (1:1, 4.8 mL) and then dried at 60°C under reduced pressure to thus obtain (2S,4S)-1-[2-[(4-ethoxycarbonylbicyclo[2.2.2]oct-1-yl)amino]acetyl]-4-fluoropyrrolidine- 2-car bonitrile (yield: 1.91 g (80%)). Incidentally, the numerical values expressed in terms of "RRT" and appearing in the following Table represent relative retention times.

**[Table 2]**

| Ex. No. | Content of Impurities (%) | | | | Recovery (%) from Crude Crystals |
|---|---|---|---|---|---|
| | RRT 0.32 | RRT 2.62 | RRT 3.47 | Total Amount | |
| Ref. Ex. (crude crystals) | 1.52 | 2.08 | 0.14 | 3.74 | -- |
| 9 | N.D. | 0.07 | N.D. | 0.07 | 76 |
| 9* | N.D. | 0.16 | N.D. | 0.16 | 81 |
| 10* | N.D. | 0.13 | N.D. | 0.13 | 66 |

| | | | | | |
|---|---|---|---|---|---|
| *: Comparative Example **: HPLC Conditions A for the Measurement N.D.: not detected. | | | | | |

It was found that the (2S,4S)-1-[2-[(4-ethoxycarbonylbicyclo [2.2.2]oct-1-yl)amino]acetyl]-4-fluoropyrrolidine-2-carbonitrile prepared in Reference Example 1 mainly contains three kinds of impurities. When expressing these impurities in terms of the relative retention times (RRT values) with respect to the objective substances, they were found to be RRT 0.32, RRT 2.62 and RRT 3.47, respectively. The conventional purification methods (Comparative Examples 9 and 10) could not satisfactorily remove the impurities of RRT 2.62. However, the data listed in Table 2 clearly indicate that the impurities of RRT 2.62, whose removal has conventionally been quite difficult, can satisfactorily be removed by first reacting an acid with the aminoacetylpyrrolidinecarbonitrile derivative to form a salt and then purifying the salt of the derivative (Example 9). In other words, it has been proved that the use of the purification method according to the present invention would permit the improvement of the chemical purity of the target substance, and the simple and efficient purification of the substance at a recovery rate identical to or higher than that achieved by the conventional purification method.

### (Comparative Example 11): Synthesis of (2S,4S)-1-[2-[(4-ethoxycarbonylbicyclo [2.2.2]oct-1-yl)amino]acetyl]-4fluoropyrrolidine-2-carbonitrile L-malate:

The same procedures used in Example 3 were carried out except for using the compound prepared in Reference Example 2 and L-malic acid to give the title compound as a colorless powder (yield: quantitative).

### Purification of (2S,4S)-1-[2-[(4-ethoxycarbonylbicyclo[2.2.2]oct-1-yl)amino]acetyl]-4-fluoropyrrolidine-2-carbonitrile:

The same procedures used in Example 10 were carried out except for using the resulting L-malic acid salt to give the title compound as a colorless powder (yield: 93%). Purity: 99.67% (as determined by the HPLC technique under the measuring conditions B).

**[Table 3]**

| | Content of Impurities (%) | | | Objective Product |
|---|---|---|---|---|
| | RRT 0.93 | RRT 1.28 | Total Amount | Purity |
| 10 | N.D. | N.D. | 0.02 | 99.98 |
| 11 | 0.01 | 0.02 | 0.04 | 99.96 |
| 11* | 0.07 | 0.24 | 0.33 | 99.67 |

| | | | | |
|---|---|---|---|---|
| *: Comparative Example **:HPLC Conditions B for the Measurement N.D.: not detected. Total amount means the total amount of the impurities. | | | | |

The foregoing Table 3 shows the results obtained when purifying (2S,4S)-1-[2-[(4-ethoxycarbonyl-bicyclo-[2.2-2]-oct-1-yl)-amino]-acetyl]-4-fluoropyrrolidine-2-carbonitrile prepared in Reference Example 2, using hydrochloric acid (Example 10), maleic acid (Example 11) and L-malic acid (Comparative Example 11). When expressing the impurities contained in the objective product in terms of the relative retention times (RRT) with respect to the objective product (HPLC Conditions B for the Measurement), they were found to be RRT 0.93 and RRT 1.28, respectively. The data listed in Table 3 clearly indicate that in Examples 10 and 11, the impurities of RRT 0.93 and RRT 1.28 which cannot satisfactorily be removed in Comparative Example 11 are sufficiently removed and that the procedures used in Examples 10 and 11 thus permit the achievement of a high purification effect. In other words, it is clear that the use of the purification method according to the present invention would permit the simple and efficient removal of these impurities whose removal had been quite difficult and that the method permits the production of (2S,4S)-1-[2-[(4-ethoxycarbonylbicyclo[2.2.2]oct-1-yl)amino]acetyl]-4-fluoropyrrolidine-2-carbonitrile having a high purity of not less than 99.9%.

### (Comparative Example 12): (2S,4S)-1-[2-[(4-ethoxycarbonylbicyclo[2.2.2]oct-1-yl) amino]acetyl]-4-fluoropyrrolidine-2-carbonitrile malonate:

The same procedures used in Example 4 were carried out except for using the compound prepared in Reference Example 2 and malonic acid to give the title compound as a colorless powder (yield: 589 mg (91%)). As a result, it was found that impurities whose formation was not observed or detected in Examples 10 and 11 were produced as by-products (about 0.5%).

### Industrial Applicability

The present invention can provide a method for simply and efficiently purifying an aminoacetylpyrrolidinecarbonitrile derivative represented by the general formula 1, which is useful as a DPP-IV inhibitor and accordingly the present purification method is industrially useful.

## Claims

1. A method for purifying an aminoacetylpyrrolidinecarbonitrile derivative comprising steps of:
(1) a step of acting maleic acid, an organic sulfonic acid or an inorganic acid on an aminoacetylpyrrolidinecarbonitrile derivative represented by the following general formula 1 to thereby form a salt of the aminoacetylpyrrolidinecarbonitrile derivative: 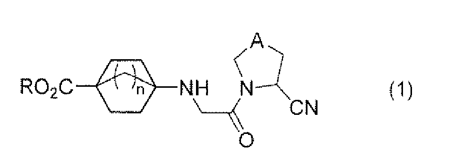 wherein
A represents CH₂, CHF or CF₂;
R represents a C₁ to C₆ alkyl group which may have a substitutent, a C₃ to C₈ cycloalkyl group which may have a substitutent, an arylmethyl group which may have a substitutent, an arylethyl group which may have a substitutent, an aromatic hydrocarbon ring which may have a substitutent, an aromatic hetero ring which may have a substitutent, or an aliphatic hetero ring which may have a substitutent; and
n represents 1 or 2;
(2) a step of isolating the salt of the aminoacetylpyrrolidinecarbonitrile derivative; and
(3) a step of treating the isolated salt of the aminoacetylpyrrolidinecarbonitrile derivative with a base to thus bring the salt back to the aminoacetylpyrrolidinecarbonitrile derivative.

2. The method for purifying an aminoacetylpyrrolidinecarbonitrile derivative as set forth in claim 1, wherein the organic sulfonic acid is methanesulfonic acid, ethanesulfonic acid, propanesulfonic acid, 2-propanesulfonic acid, benzenesulfonic acid, 4-toluenesulfonic acid or naphthalenesulfonic acid.

3. The method for purifying an aminoacetylpyrrolidinecarbonitrile derivative as set forth in claim 1, wherein the inorganic acid is hydrochloric acid, sulfuric acid, hydrobromic acid or phosphoric acid.

4. The method for purifying an aminoacetylpyrrolidinecarbonitrile derivative as set forth in claim 2, wherein the organic sulfonic acid is methanesulfonic acid.

5. The method for purifying an aminoacetylpyrrolidinecarbonitrile derivative as set forth in claim 3, wherein the inorganic acid is hydrochloric acid.

6. The method for purifying an aminoacetylpyrrolidinecarbonitrile derivative as set forth in any one of claims 1 to 5, wherein A is CHF, R is an ethyl group and n is 2.

7. A salt of an aminoacetylpyrrolidinecarbonitrile derivative represented by the following general formula 2: 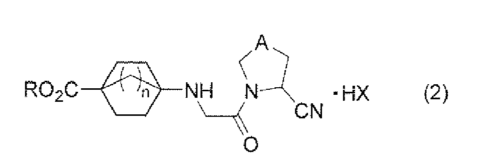 wherein
A represents CH₂, CHF or CF₂;
R represents a C₁ to C₆ alkyl group which may have a substitutent, a C₃ to C₈ cycloalkyl group which may have a substitutent, an arylmethyl group which may have a substitutent, an arylethyl group which may have a substitutent, an aromatic hydrocarbon ring which may have a substitutent, an aromatic hetero ring which may have a substitutent, or an aliphatic hetero ring which may have a substitutent;
n represents 1 or 2; and
HX represents maleic acid, an organic sulfonic acid or an inorganic acid.

8. The salt of an aminoacetylpyrrolidinecarbonitrile derivative as set forth in claim 7, wherein HX represents maleic acid, methanesulfonic acid or hydrochloric acid.

9. The salt of an aminoacetylpyrrolidinecarbonitrile derivative as set forth in claim 7 or 8, wherein A is CHF, R is an ethyl group and n is 2.

10. An aminoacetylpyrrolidinecarbonitrile derivative represented by the following general formula 1 and **characterized in that** the derivative has an impurity content of less than 0.1%: 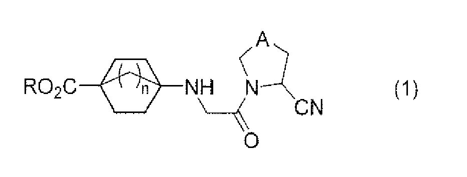 wherein
A represents CH₂, CHF or CF₂;
R represents a C₁ to C₆ alkyl group which may have a substitutent, a C₃ to C₈ cycloalkyl group which may have a substitutent, an arylmethyl group which may have a substitutent, an arylethyl group which may have a substitutent, an aromatic hydrocarbon ring which may have a substitutent, an aromatic hetero ring which may have a substitutent, or an aliphatic hetero ring which may have a substituted; and
n represents 1 or 2.
